# EUROPEAN PATENT APPLICATION

(11) **EP 4 749 630 A1**
(43) Date of publication of application: **27.05.2026**
(21) Application number: 25217249.9
(22) Date of filing: 20.11.2025
(51) Int. Cl.: G16H 10/00, H04L 1/00, H04L 65/75

(54) **INVALID DATA STREAM IN A MULTI-SYSTEM INTERACTION**

(30) Priority: 20.11.2024 US 202418954237
(71) Applicant: Cilag GmbH International, 6300 Zug (CH)
(72) Inventor: MUELLER, Karl W., Cincinnati, 45242 (US); ADAMS, Shane R., Cincinnati, 45242 (US); COWPERTHWAIT, Matthew David, Cincinnati, 45242 (US); ROSS, Nicholas James, Cincinnati, 45242 (US); SHELTON IV, Frederick E., Cincinnati, 45242 (US); JONES, Shannon L., Cincinnati, 45242 (US); BRADY, John E., Cincinnati, 45242 (US)
(74) Representative: Carpmaels & Ransford LLP

(57) **Abstract**

A surgical system may include a processor configured to make a determination that a data stream is invalid. The processor may be further configured to obtain a data stream associated with a measurement from a surgical device. The processor may be further configured to generate a first control signal associated with the surgical device based on the data stream. The processor may be further configured to detect that the data stream is invalid. Upon detecting that the data stream is invalid, the processor may be further configured to determine an approximation factor associated with the data stream. The processor may be further configured to generate a second control signal associated with the surgical device based on the determined approximation factor.

## Description

### CROSS-REFERENCE TO RELATED APPLICATIONS

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
- U.S. Patent Application No. 18/954,186, filed November 20, 2024, entitled METHOD FOR MULTI-SYSTEM INTERACTION, and
- U.S. Patent Application No. 18/954,228, filed November 20, 2024, entitled CONFLICTING DATA STREAMS IN MULTI-SYSTEM INTERACTION.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Systems, methods, and instrumentalities are disclosed herein for a surgical system. A surgical system may include a processor. The surgical system may obtain a data stream associated with a measurement from a surgical device. The surgical system may generate a first control signal associated with the surgical device based on the data stream. The surgical system may detect that the data stream is invalid. Upon detecting that that the data stream is invalid, the surgical system may determine an approximation factor associated with the data stream. The surgical system may generate a second control signal associated with the surgical device based on the determined approximation factor.

For example, the surgical system may introduce a perturbation to an input signal of the surgical device. The surgical system may receive a value in the data stream upon introducing the perturbation. The surgical system may determine an expected value in the data stream in response to the perturbation. The surgical system may compare the received value to the expected value. The surgical system may assess a validity of the data stream based on the comparing to monitor the data stream.

The surgical system may introduce a perturbation to an input signal of the surgical device. The surgical system may determine an expected control value in response to the perturbation. The surgical system may determine a difference between the expected control value and a normal control value. The approximation factor associated with the data stream may be determined based on the difference between the expected control value and the normal control value. The surgical system may adjust a response of the surgical device based on the approximation factor.

The data stream may be determined to be invalid based on an expected range associated with the measurement. Based on detecting a measured value in the data stream being outside of the expected range associated with the measurement, data stream may be determined to be invalid.

The surgical system may generate a corrected data stream associated with the measurement based on the approximation factor and the data stream. The second control signal associated with the surgical device may be generated based on the corrected data stream.

The surgical system may transform the data stream based on the approximation factor. The second control signal associated with the surgical device may be generated based on the transformed data stream.

The surgical system may detect a measurement difference between the data stream and historic data. The surgical system may compare the measurement difference to an error tolerance threshold value. The detecting that the data stream is invalid may be based on the measurement difference satisfying the error tolerance threshold value.

Determining the approximation factor may further include identifying the surgical device that generates the data stream. Determining the approximation factor may further include obtaining an initial characterization of the surgical device. The approximation factor may be determined based on the initial characterization of the surgical device.

The surgical system may detect a measurement difference between the data stream and historic data. The surgical system may identify a disagreement between the data stream and the historic data. The surgical system may determine a cause of the identified disagreement, and the approximation factor may be determined based on the cause of the identified disagreement.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 shows an example surgical instrument.
FIG. 6 shows an example computer-implemented surgical system for determining a data stream is invalid.
FIG. 7 shows an example flowchart for determining a data stream is invalid.

### DETAILED DESCRIPTION

A more detailed understanding may be had from the following description, given by way of example in conjunction with the accompanying drawings.

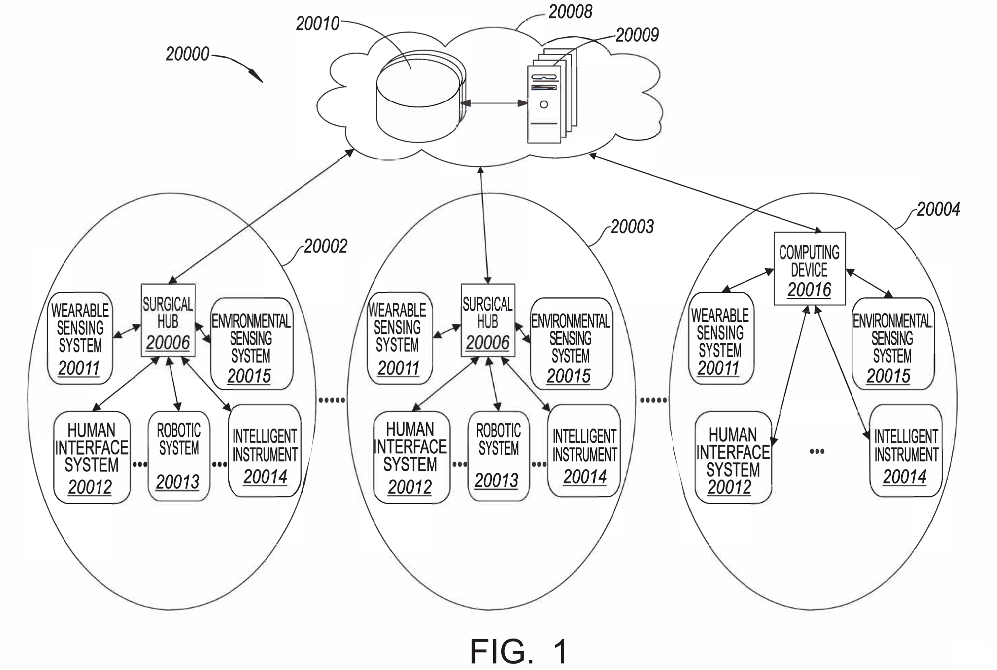

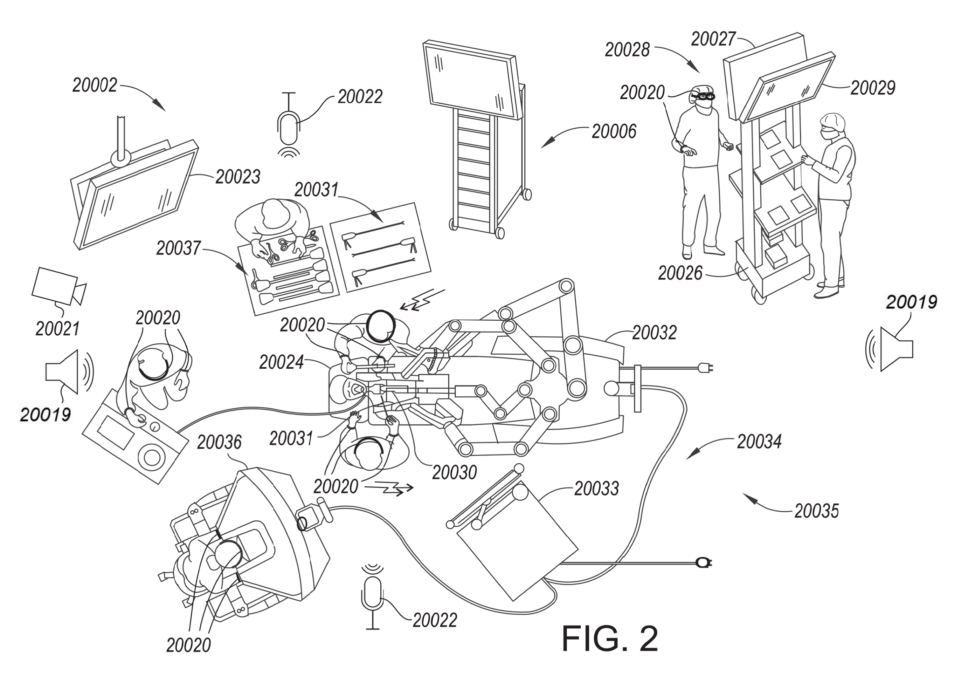

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include, tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

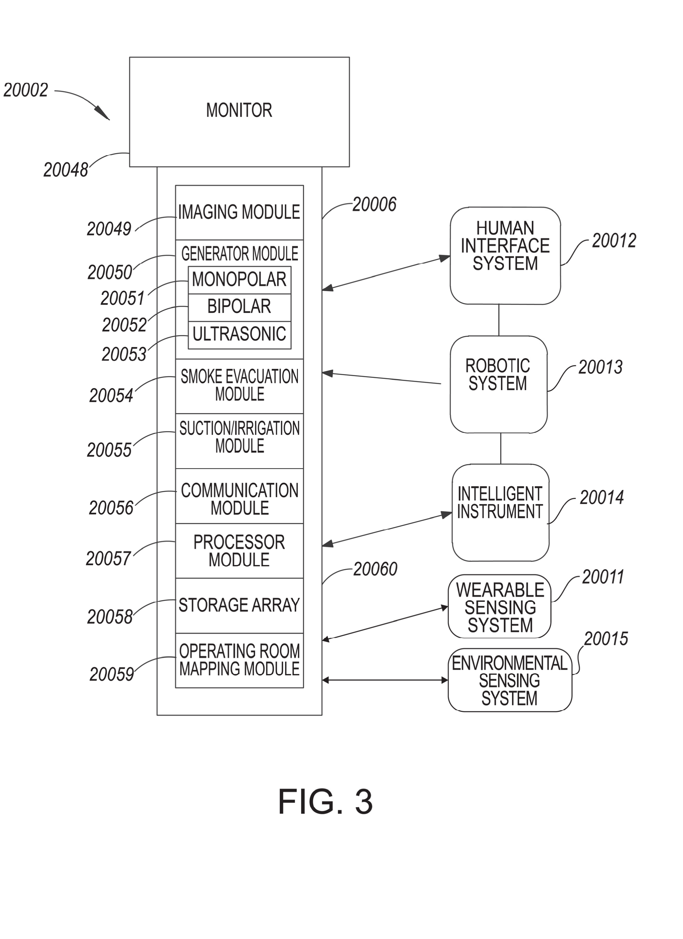

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using, ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

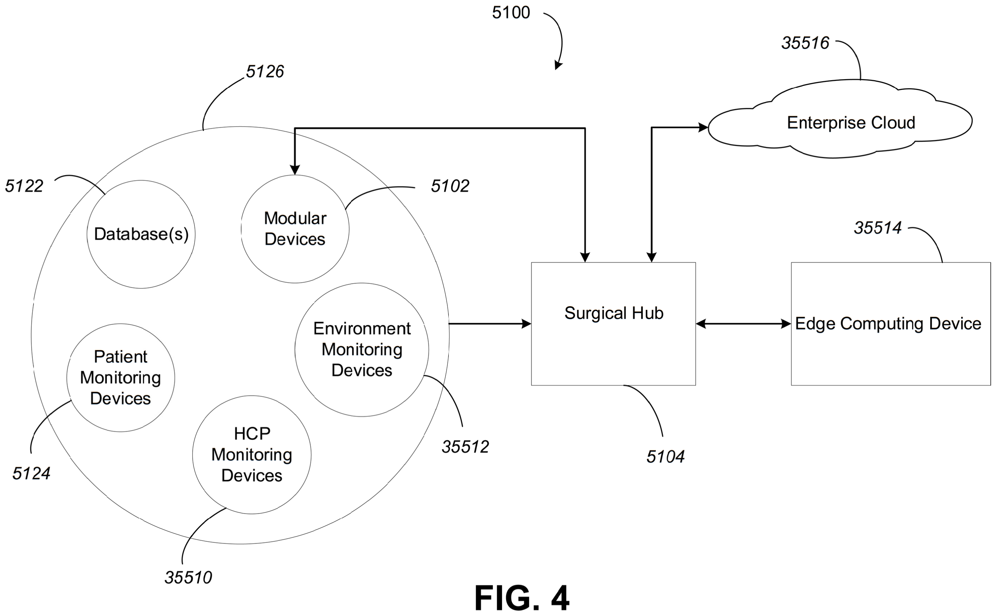

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

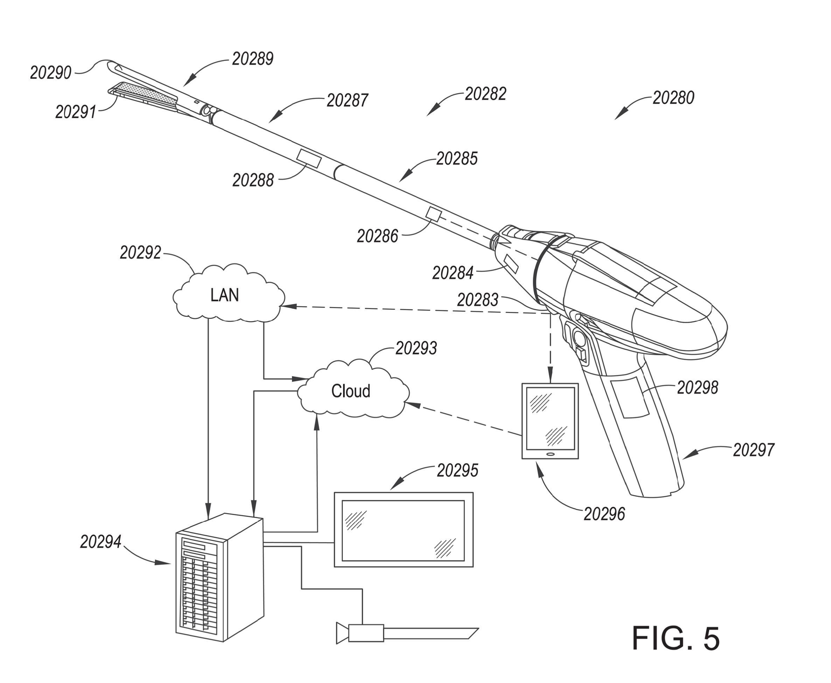

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

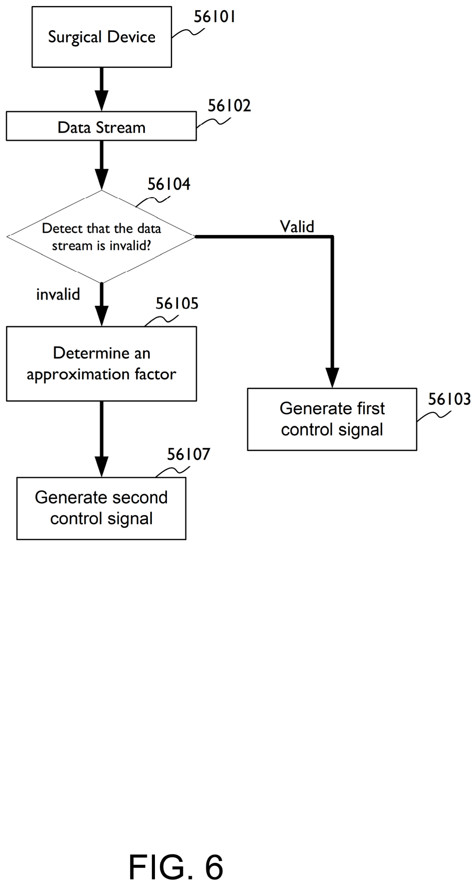

FIG. 6 shows an example computer-implemented method for determining whether a data stream is invalid and generating a control signal. For example, a surgical system may include or may interact with a surgical device 56101. The surgical device 56101 may output a data stream 56102 associated with a measurement. In some examples, the data stream 56102 may provide inaccurate data. At 56104, whether the data stream is invalid may be determined. Based on detecting that the data stream is valid, a first control signal may be generated at 56103. Based on detecting that the data stream is invalid (e.g., determining that the data stream is invalid), at 56105, an approximation factor may be determined. The approximation factor may be used to generate a second control signal at 56107. In some examples, the approximation factor may be combined with (e.g., factored into) the first control signal to generate a second control signal. The first and/or the second control signal may be sent to a surgical device as described herein, a surgical instrument as described herein, or a computing system, such as a surgical hub 20006.

In some examples, the data stream generated from the surgical device may be a single data stream. The data stream may be determined to be invalid and/or misleading the control system to an undesirable outcome. When the data stream is determined to be invalid and/or misleading the control system to an undesirable outcome, the surgical system may identify the issue (e.g., the type of issue), make a correction, and/or determine that the surgical system needs additional information (e.g., sources of information).

The surgical system may identify the (e.g., the type of issue), for example, whether the issue is related to a physiologic change in patient status that invalidates a previously valid data stream and/or whether the issue is related to a signal and/or electrical cause.

In examples, the validity of a data stream may be assessed (e.g., a data stream validity assessment) via intentional disruptions. Intentional perturbations may be introduced into the data stream to assess the validity of the data stream, for example, to assess if the data stream reacts as expected. For example, using an intentional perturbation may show lag in the system or a lack of response. In a closed loop system, an intentional insertion of incorrect data (e.g., an intentional perturbation) into a data stream may be used to monitor a response and adjust the control signal.

The introduced perturbation to an input signal of the surgical device may include data that is testing a known or unknown issue of the surgical system and/or surgical device. The value in the data stream may be received upon introducing the perturbation. An expected value in the data stream may be determined in response to the perturbation. The surgical system may then detect if the data stream is invalid if the perturbation does not cause the expected value to be returned in the data stream.

A single data stream may be misleading the control system to an undesirable outcome. A physiologic change in patient status may invalidate a previously valid data stream. To determine if the data stream is invalid, the data stream may be compared to data at a baseline (ex. procedure initiation) or historic data to confirm measurement is within an expected range (e.g., a normal range). In examples, the data may be correct but not representative of a patient status. When comparing the generated data to the range, a threshold (e.g., an error tolerance threshold value) may be applied to allow for discrepancies. The threshold may be a range, boundary, and/or limit, for example, when determining the validity of a measurement. For example, historic data of a patient may show the average body temperature of the patient within a range. A measured value from a data stream of the temperature of the patient may be determined to be valid if the measured value is within the range of the average body temperature of the patient. In examples, a boundary may be determined at a minimum and/or maximum heartrate. If a measured value from a data stream of the heartrate of the patient is determined to be below the minimum heartrate boundary or above the maximum heartrate boundary, the measured value may be determined to be invalid and alert an HCP. A measurement difference may be determined in addition to determining the validity of a measured value, The measurement difference may highlight the variance of the measured value from the expected value in a data stream. In examples, historic data of a patient may show the average body temperature of the patient within a range. A measured value from a data stream of the temperature of the patient may be significantly below the range of the average body temperature of the patient. In this example, the HCP may take more immediate action to this larger discrepancy. A cause of the identified discrepancy and/or disagreement may be identified. The identified cause of the disagreement may allow the HCP more information for making a decision.

In examples, the cause of the data stream to be invalid may be due to signal and/or electronic malfunctions. Examples of signal and/or electronic malfunctions include interference, signal loss, cross-talk, a distorted and/or weak signal, delay and/or latency, and signal reflection. Interference may include disruption caused by external electromagnetic signals, such as from radios, cell phones, or power lines. Signal loss may include a loss of signal due to low-quality connectivity, physical damage to cables, or other obstructions. Cross-talk may include signals from one communication channel affecting another, causing confusion and/or miscommunication. A distorted signal may include deformation of a signal during transmission, for example, due to noise, faulty hardware, or signal processing. A weak signal may include a reduced signal strength, leading to low-quality reception or functionality. Delay and/or latency may include unintended delay in signal transmission affecting communication, internet speeds, or synchronization in data streams. Signal reflection may include a signal bouncing back to the source or to other devices, causing unintended duplication or overlap.

In examples, a cable detection signal or system may be used to discriminate between hardware issues and potential patient issues. For example, a ground and/or signal interlock may be used. The cable or mechanism may ground out a specific pin on the peripheral for determining if the pin has been disconnected.

In examples, a loopback interlock may be used. The system may provide a loopback mechanism. The capital equipment may provide a signal. The signal may pass through the cable and any peripheral equipment, before returning back to the capital system where it is monitored. If the return signal is not received, then it may be assumed that the signal is lost or corrupted due to an error in the hardware or connection itself.

In examples, active cable pulsing may be used. The cable may generate a specified tone (such as a 1kHz sine or square wave, for example) in the background, to either be passed on the same wire as the monitored signal, or a distinct wire as the monitored signal (or combination of both). If this signal is not detected by the capital equipment, then it may be an indication that the monitoring peripheral has been disconnected or is faulty.

In examples, a sensing heartbeat system may be used. In a sensing heartbeat system (e.g., smart sensing heartbeat system), the peripheral may send a periodic heartbeat message such as at 1 Hz intervals to broadcast its current status, and that its connectivity status to the surgical system. In examples, a smart system status may be used. For a smart system, the capital equipment or controlling system may query the connected peripheral for its status. The status information of the smart system may be sent (e.g., always be sent) when other pertinent data is sent.

In examples, a calibration jig may be used. A calibration system or setup may be provided. The equipment and/or surgical device(s) may be connected to the calibration system. The connection of the equipment and/or surgical device(s) to the calibration system may produce a known response at different points of the equipment. For example, a pulse oximeter may have a 'dummy finger' to provide a predetermined cadence to it. The dummy finger of the pulse oximeter may allow measurements to confirm the system is working correctly.

In examples, in-series sensing system with independent monitoring may be used. The system may provide a mechanism within its own monitoring capabilities to allow a second system to independently monitor it without corrupting or impacting the monitoring of the primary system.

In examples, a voltage readback source may use a calibrated shunt resistor to allow for voltage readings of current (with a known resistance) from a second source. Since both the voltage taps of the shunt resistor and the resistance are known, the system may additionally infer current. The overall impact may be negligible to the primary system. This may allow a second potential monitoring point for a separate system in the event of an error with the primary system.

In examples, bounding of a signal range may be used. A sensor may have the capability to provide a signal over a greater range than is physiologically relevant or capable. As a result, the system may use software or other intelligence to monitor if the signal has exceeded the physiological range and if it is in a zone that may indicate a potential error.

In examples, a sensor may have a range of temperature it can provide back from 0 - 5V, for example. However, that range of temperature may correlate to 0 degrees Celsius (°C) to 125°C. We know that the human body will typically remain around 36°C, or about 1.44V on the sensor. We know that if the temperature deviates more than 10°C in either direction, it may be (e.g., is likely) an indication of a bad reading. The corresponding voltage from the temperature data may correlate to 26°C (1.04V) or 46°C (1.84V). As a result, the system may implement thresholds at 1V and 2V to use as a range for a valid signal. Anything outside of those voltages may indicate a signal error.

Incorrect data may be compensated to enable continued system control to correct data stream. In examples, if a data stream is detected and/or determined to be invalid, the data stream may be compensated and/or corrected to be sent to the surgical system.

The surgical system may make a correction to the data stream(s) (e.g., erroneous data stream(s)) to correct for a wrongly corrected data stream. A transformation of the incorrect data stream may be made based on risk associated with the data stream, the surgical device, the measurement, etc. For example, if a measurement is associated with a higher risk of the health of the patient, the transformation associated with that risk may be reduced to avoid exposing the patient to that risk.

Correction factors may be utilized to adjust the signal to correct for a wrongly corrected data stream. Adjustment of the surgical system may be based on an initial characterization of the surgical system. In examples, a system transmitting temperature may be given as a numerical value, without units attached to it. They are transmitted in °C, but the receiving system expects the temperature to be received in Fahrenheit. As a result, the data stream may be viewed by the system as technically incorrect, although the data may be still seen as valid. The data may be assessed to be in the wrong unit.

Transformation or adjustment(s) of the acceptable boundaries of a data stream may be implemented to correct for a wrongly corrected data stream. If a data stream is incorrect, the boundaries of the data stream may be corrected to include a wider and or narrower range, for example. Responses to the system may be trend based or magnitude based, for example. Data streams that have a variation and/or are still performing consistently, even if the value is incorrect in absolute terms, may have their trending adjustment be implemented.

For example, an approximation factor may be a multiplier that is applied as a transformation to the data stream to generate a corrected data stream. The approximation factor may be an equation that is applied to the data stream to generate a corrected data stream. In an example, an approximation factor may be an equation that generates a data stream in degrees Celsius from a data signal that was received in degrees Fahrenheit. In examples, a patient temperature may be determined to be low using a patient temperature monitor. An initial characterization of the patient temperature monitor may be obtained which shows that the patient temperature monitor is reading the temperature of the patient in degrees Celsius while the data is being interpreted in degrees Fahrenheit. An approximation factor may be applied to the temperature being read in degrees Celsius to alter the data to correspond with degrees Fahrenheit.

Incorrect data streams may be ignored based on risk (e.g., risk associated with the health of the patient). For example, if an incorrect data stream is associated with a higher risk of the health of the patient, then the data stream associated with that risk may be ignored to avoid exposing the patient to that risk.

Intentional calibration of the surgical system may be implemented to correct for a wrongly corrected data stream. Intentional calibration of the surgical system may include white light balancing and/or temperature calibration by a user. In examples, temperature sensors may be applied to the patient. A user (e.g., a surgeon) may select to normalize the surgical system. The system may normalize certain values to associated 100%. It may not matter as a result if the temperature received is received in degrees Fahrenheit or degrees Celsius, as the system could look at the difference in trending or percent difference.

Indirect calibration on realistic bounding or norms may be implemented to correct for a wrongly corrected data stream. In an example, temperature sensors may read in the range of 18 - 30 degrees. This range may be unreasonable for most operating rooms in degrees Fahrenheit, but perfectly reasonable for degrees Celsius. As a result, the system may make an interpretation that the temperature being received is in degrees Celsius.

The surgical system may determine that the surgical system needs additional information (e.g., sources of information). In examples, the surgical system may be providing a data stream on a probe location. The probe depth within the lung may reduce the accuracy of the location data stream. Additional input or a new data stream may be needed to locate the probe position.

The surgical system may lose confidence in position over time. This may result in the surgical system being unable to reconcile a control signal from the collected data. Trigger events may increase uncertainty of probe position and/or location including but not limited to monopolar activation or a CT machine in the field. Consistent events are tolerable but transient events may be catalogued.

Establish library of events to categorize and communicate events that trigger data uncertainty. Electromagnetic drift against kinematic movement may result in an absolute change and/or a percentage change. Percentage errors in changes in positioning system may be compared to recorded changes in kinematic movement.

A relative error assessment may be implemented for the system triggers when indicating insufficient control data. The system may continually calculate perceived confidence in a location. The perceived confidence may be provided back to the user (e.g., surgeon).

A linear assessment against pre-surgical scans may be implemented for the system triggers when indicating insufficient control data. Computation of forward kinematics against the pre-surgical scans may be used to determine confidence levels, for example.

The surgical system may determine a location is drifting independently. In examples, electromagnetic sensors may be laddered to determine a location is drifting. Laddering of linked electromagnetic sensors, where each sensor can provide data of its current location, may allow a stackup to be performed of the location within the body, and thereby reduce error.

Alternative mechanisms may be utilized for a secondary determination of the position of a device. Alternative mechanisms for a secondary determination of the position of a device may include methods that do not include the use of a Cone Beam CT or surgical system existing electromagnetic navigation system to determine its current location.

Alternative mechanisms for a secondary determination of the position of a device may include utilization of a radioactive or contrast material to help determine the absolute location and be utilized as a beacon.

Alternative mechanisms for a secondary determination of the position of a device may include alternative frequency of communication that is less impacted by the body. In examples, low frequency beacons (such as below 100 MHz) may have better mechanisms of passing through the body, and may be able to determine the location via signal strength, such as triangulation of the signal.

Alternative mechanisms for a secondary determination of the position of a device may include bright light (visible and/or infrared). A high power light may be blinked on for an extremely short duration to avoid thermal injury, but could be picked up and located by multiple cameras in the room that are synchronized to flashing of the light.

Alternative mechanisms for a secondary determination of the position of a device may include an EM sensor in an induced EM field utilized for signal strength to triangulate position. To improve tracking, multiple EM sensors may be placed at fixed intervals along a flexible tube. Each sensors may report its individual position(s), which may be correlated to the previous and/or subsequent sensor (or "local pair") on the shaft using the fixed distance along the scope as a reference length. By chaining each sensor to its local pair, an overall vector map of the shaft's position and orientation can be reconstructed in space, providing higher fidelity positional tracking and context to the operator. Using a point and its local pair, the system may quantify positional error (or measurement drift) by calculating spatial separation and referencing against the fixed shaft distance. Stacking error from point to point may provide a map of interference in critical areas of operation, potentially allowing for measurement drift compensation, or to calculate a "confidence" value which can be communicated to the operator. An additional embodiment may compare the current detected position to the last known position and/or the "change in commanded position" by the surgical system/s platform. This may be performed on each point to estimate error along the whole shaft.

Alternative mechanisms for a secondary determination of the position of a device may include sound and/or a microphone. The surgical device may use EM waves to locate the beacon on the tip of the endoscope. When the scope is deep within the body cavity, the accuracy of the position may be diminished. A CT scanner may be brought in to act as a secondary source of localization. However, it may interfere with the surgical system's readings. Multiple signals may be used that communicate the same information through different forms as a secondary check while preventing against interference and other issues. In examples, the surgical system may utilize a speaker in the endoscope tip combined with a microphone array in the operating room. During the procedure, the speaker may emit a tone or series of tones at a set frequency (ultrasonic and/or subsonic to prevent the operating room staff from hearing. The microphone array may be set up in a known position around the patient and designed to detect the set frequency of the speaker. Using an array allows for triangulation or other methods of position detection. In addition to an array in the operating room, microphones may be placed at a known position in natural orifices close to the endoscope (e.g., the esophagus), which could improve accuracy. The frequency or sound emitted may need to be chosen so it can penetrate the human body cavity. Since sound acts through a different medium than EM waves, it would have a lower likelihood of interfering with the surgical system position detection while simultaneously providing a secondary check. This same concept of multi-signal inputs may be used for other Interactive Smart Systems in the operating room by utilizing different forms, similar to humans having multiple senses. Utilization of a radioactive or contrast material may help determine the absolute location and be utilized as a beacon.

Alternative mechanisms for a secondary determination of the position of a device may include laparoscopically assisted solutions. Laparoscopically assisted solutions may include a camera for lighting, a magnetic sensor, for example. A separate laparoscopic incision may be used to insert a magnetic sensor, that may exist on the outside of the lung area, but may be used to help confirm the location of the surgical system sensor.

Alternative mechanisms for a secondary determination of the position of a device may include on-patient markers detected by the surgical system and/or may be compared to CT. To improve accuracy of the EM position sensing, a 2D calibration grid may be utilized. A 2D grid of EM sensors may be fixed on the table at known distances and locations from each other. The grid would remain in the same physical location during the course of the procedure. The surgical system may continuously monitor the location of these non-moving, known locations to their true positions. The system may optionally apply the appropriate offset to the system if the position of the sensors displays out of a determined tolerance due to changes to the EM field.

The surgical system may incorporate a new data stream. The surgical system may be able to run with present data. The surgical system may indicate additional data is needed. The surgical system may add additional data from one of the systems. The surgical system may know what other information is needed and/or idea but may have limited bandwidth (e.g., a surgical system may use minimum viable data).

Signal loss or corruption of flexible endoscopic robot position data, for example, may also be provided. The surgical system may monitor data latency and latency variation (e.g., jitter) during a procedure in order to identify when the latency or jitter exceeds a pre-defined threshold at which it has been determined that surgeon performance may suffer.

On-patient markers may be detected by the surgical system and/or compared to a CT. To improve accuracy of the surgical system position sensing, a 2D calibration grid may be utilized. A 2D grid of EM sensors may be fixed on the table at known distances and locations from each other. The grid would remain in the same physical location during the course of the procedure. The surgical system could continuously monitor the location of these non-moving, known locations to their true positions. The system then may optionally apply the appropriate offset to the system if the position of the sensors displays out of a determined tolerance due to changes to the EM field.

A 2D calibration grid for the surgical system position accuracy may include using multiple interactive smart systems to locate oneself within the body. Issues may arise due to difficulties knowing up from down, left from right, on the live endoscope camera, for example. This may force the user (e.g., the surgeon) to rely mainly on known landmarks from CT scans or ultrasounds, which may often be difficult to find. A calibration system may be used at the outset of a procedure to set a global coordinate system that may project onto the endoscope camera live feed. Other smart system screens may remedy this issue.

A 3D calibration grid may be used to aid in endoscope camera orientation. Markers may be placed on the patient pre-operation that may be used for triangulation. They may need to be sensitive to CT and EM energy. At the beginning of a procedure a CT may be performed to relocate the tumor. The markers may be geolocated on the CT image. Measurements may be taken from the markers to the tumor. The CT may be shut off and the EM machine may then be used to sense the markers on the patient. This may map out the location of the markers for the EM sensor. The EM and CT data may be overlayed to calibrate how the EM measurements relate locationally to the CT data. This may be used in a feedback loop with the endoscope to know where the endoscope tip is in relation to the tumor. During the procedure, if locational data is unable to be obtained due to EM sensor dilution, an error may be displayed to the surgeon to let them know the location of the endoscope is not within a pre-defined margin of error, suggesting need for secondary location feedback.

Self-latency monitoring of overlaid laparoscopic video before or during a surgical procedure may be provided. The surgical system may monitor video and/or device data latency and/or latency variation (e.g., jitter) during a procedure to identify when the latency or jitter exceeds a pre-defined threshold at which it has been determined that surgeon performance may suffer. In examples, thresholds may appear to be approximately 160ms for a total system video latency and approximately 30ms for a system video jitter.

The system may monitor video and/or device data latency and/or latency variation (e.g., jitter) before the procedure, during a system setup, to allow the surgeon to decide whether or not to proceed with the procedure as planned, or to directly connect the laparoscopic camera system to the surgical monitor (e.g., via a video router), bypassing the surgical system.

The surgical system may mitigate latency and jitter (e.g., excessive latency and jitter) in the event the clinically acceptable thresholds are exceeded. The surgical system may display a notification and/or alert on the surgical monitor overlay informing the surgeon that the acceptable latency and/or jitter threshold has been exceeded. An LED (or similar) indicator on the surgical system or associated hardware may be illuminated. An audible tone may be annunciated by the surgical system, either alone or in conjunction with other notification methods. This tone may be sufficiently loud to be heard above typical levels of background noise in the operating room. The surgical system may re-route the video output so that it bypasses the Hub (directly from laparoscopic camera to surgical monitor). The surgical system may display the real-time latency and/or jitter measurements on the surgical monitor overlay, possibly drawing additional attention to them when they exceed the clinically acceptable thresholds. The surgical system may disable video output to the surgical monitor (possibly with a delay) to ensure the surgeon is not operating using the surgical system in a situation with unacceptable latency or jitter. The surgical system may prioritize certain tasks that may reduce overall system latency. In examples, if video recording is in process, this may be paused since it may not be essential to patient health. In the event of excessive surgical device data latency, the software may display a notification or alert on the surgical monitor informing the surgeon to refer to the surgical device itself for timely information, alerts and alarms.

In examples, the surgical system may have 8 cores and support 16 threads. One of these cores/threads may be dedicated to monitoring latency and jitter so as not to impact the performance of other functions. Latency may be measured based on video and/or data input to the system and video output from the system so just the surgical system's contribution to the overall latency is measured. Time zero (t0) may be defined (for video) as when the video frame grabber or FPGA receives/grabs the video frame and the end-time (tf) may be defined as when the GPU processes/outputs the overlaid frame. Jitter may be defined as the difference between consecutive tf - t0 intervals. For device data, t0 may be defined as when the raw device data message is received which is logged and tf may be defined as for the video latency scenario above.

A spare video input and output of the digital hub may be used to assess latency prior to a surgical procedure. This may be achieved by the surgical system timestamping an incoming video frame from the laparoscopic camera, outputting this frame on a spare video output, and the re-reading the same frame (timestamped) on the spare video input. The difference between the timestamps associated with the successive input frames may closely approximate the latency introduced by the surgical system. By monitoring laparoscopic video latency and latency variation (jitter) prior to and/or during a surgical procedure, the surgical system may identify when these latency measures exceed a clinically acceptable threshold and take action to mitigate any resulting potential harm to the patient. Surgeons may benefit by being alerted of excessive video latency and latency variation before it affects the surgical procedure and leads to patient harm. By monitoring surgical device data latency prior to and/or during a surgical procedure, the surgical system may identify when these latency measures exceed a clinically acceptable threshold and may take action to mitigate untimely surgical device information display on the surgical monitor and any resulting surgeon frustration.

Excessive laparoscopic video latency and video latency variation (e.g., jitter) from the laparoscopic camera to the surgical monitor may adversely impact surgeon performance and lead to patient harm. Excessive surgical device (e.g. electrosurgical generator or surgical instrument) data latency may result in delayed display of relevant device information on the surgical monitor and may result in delayed surgical procedures and surgeon frustration.

A lack of patient information may be resolved at procedure initiation. In an example with an unknown patient, 16% of procedures are emergency compared to planned, and of these, many are time-sensitive and preoperative planning and imaging are not possible. Smart systems may deal with a lack of information. In examples, for a patient with excessive internal bleeding, an ultrasonic evaluation, CT, MRI may not have visibility due to the presence of blood and tissue. A database history may be reviewed. A WBC count may be elevated. When a patient identified as unknown0, a "tag" may be started on them to intentionally collect data from beginning of care to build a history of them compared to big dataset. Unknowns about the patient may be reduced.

Real-time data may inform the next steps to be taken. Patient factors may be determined based on inspection. An analysis of the patient may inform the system on an approach. Demographic data may be available that may indicate a risk profile of the patient. The data may be fed into simulation to approximate the patient risk profile. In examples, in the context of respiration, the heartrate and/or breathing may change erratically or be depressed. If the patient had previous lobectomy, the ventilator may indicate that inhalation and/or exhalation volume representation has a reduced volume. Weight and/or height of the patient may be combined with estimated tidal volume. Scars and/or past procedure markings on surface of the patient may be noted. In the context of imaging, if there is a puncture trauma to a lung lobe, the smart system may indicate regions that are impacted. For heart stents, if the HCP does not have time to run a CT, additional implantables may be detected within the patient.

Biomarkers may be used to trace a patient. Biomarkers available include the retina and thumb print. The system may store de-identified data. Patient records may be found to be most similar to the patient in the OR. Biomarkers may be stored (e.g., keep them separated from PHI information) and may enable the surgeon to confirm the identity of the patient.

A curve (e.g., distribution curve) may be fit to other patients in the OR. Information including, but not limited to, surgical history, patient history, allergies, current medication, and previous disease history, may be needed but not available through a real-time test. When there is insufficient preoperative imaging, intraoperative imaging may supplement. Local site imaging may be communicated to other smart devices using the feed to provide context or orientation origin. Rates of unplanned re-operation have been reported as highly variable in the literature, ranging from 0.8% to 7%. Evaluating and tracking unplanned surgical results on surgical wards may raise awareness of complications and surgical errors.

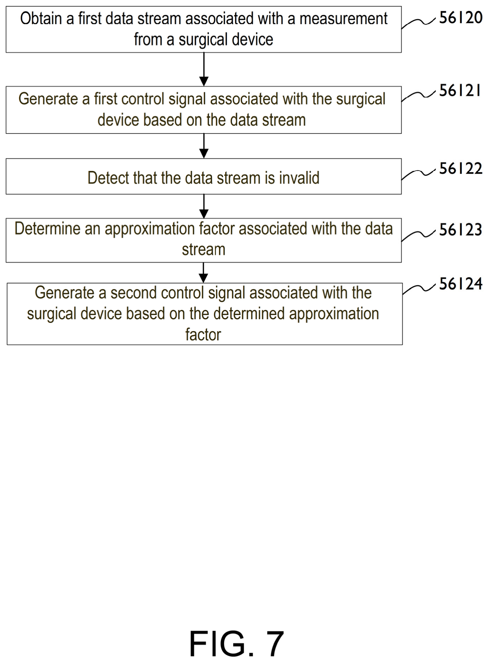

FIG. 7 shows an example flowchart for determining a data stream to be invalid and generating a control signal based on a determined approximation factor. At 56120, a first data stream may be obtained. At 56120, the first data stream may be associated with a measurement from a surgical device. At 56121, a first control signal associated with the surgical device may be generated based on the data stream. At 56122, the data stream may be detected and/or determined to be invalid. At 56123, an approximation factor associated with the data stream may be determined. At 56124, a second control signal associated with the surgical device based on the determined approximation factor may be determined.

Invalid data streams may be detected. An approximation factor may be determined that is associated with the invalid data stream(s).

Example 1. A surgical system comprising:
a processor configured to:
obtain a data stream associated with a measurement from a surgical device;
generate a first control signal associated with the surgical device based on the data stream;
detect that the data stream is invalid;
upon detecting that that the data stream is invalid, determine an approximation factor associated with the data stream; and
generate a second control signal associated with the surgical device based on the determined approximation factor.

Example 2. The surgical system of example 1, wherein the processor is further configured to:
introduce a perturbation to an input signal of the surgical device;
receive a value in the data stream upon introducing the perturbation;
determine an expected value in the data stream in response to the perturbation;
compare the received value to the expected value; and
assess a validity of the data stream based on the comparing to monitor the data stream.

Example 3. The surgical system of any one of examples 1-2, wherein the processor is further configured to:
introduce a perturbation to an input signal of the surgical device;
determine an expected control value in response to the perturbation;
determine a difference between the expected control value and a normal control value, wherein the approximation factor associated with the data stream is determined based on the difference between the expected control value and the normal control value; and adjust a response of the surgical device based on the approximation factor.

Example 4. The surgical system of any one of examples 1-3, wherein detecting that the data stream is invalid further comprises:
obtaining an expected range associated with the measurement; and
determining the data stream comprises a measured value outside of the expected range associated with the measurement, wherein the data stream is detected to be invalid based on the data stream comprising a measured value outside of the expected range.

Example 5. The surgical system of any one of examples 1-4, wherein the processor is further configured to: generate a corrected data stream associated with the measurement based on the approximation factor and the data stream, wherein the second control signal associated with the surgical device is generated based on the corrected data stream.

Example 6. The surgical system of any one of examples 1-5, wherein the processor is further configured to: transform the data stream based on the approximation factor, wherein the second control signal associated with the surgical device is generated based on the transformed data stream.

Example 7. The surgical system of any one of examples 1-6, wherein the processor is further configured to:
detect a measurement difference between the data stream and historic data; and
compare the measurement difference to an error tolerance threshold value, wherein the detecting that the data stream is invalid is based on the measurement difference satisfying the error tolerance threshold value.

Example 8. The surgical system of any one of examples 1-7, wherein determining the approximation factor further comprises:
identifying the surgical device that generates the data stream; and
obtaining an initial characterization of the surgical device, wherein the approximation factor is determined based on the initial characterization of the surgical device.

Example 9. The surgical system of any one of examples 1-8, wherein the processor is further configured to:
detect a measurement difference between the data stream and historic data;
identify a disagreement between the data stream and the historic data; and
determine a cause of the identified disagreement, wherein the approximation factor is determined based on the cause of the identified disagreement.

Example 10. A surgical operating method comprising:
obtaining a data stream associated with a measurement from a surgical device;
generating a first control signal associated with the surgical device based on the data stream;
detecting that the data stream is invalid;
upon detecting that that the data stream is invalid, determining an approximation factor associated with the data stream; and
generating a second control signal associated with the surgical device based on the determined approximation factor.

Example 11. The surgical operating method of example 10, further comprising:
introducing a perturbation to an input signal of the surgical device;
receiving a value in the data stream upon introducing the perturbation;
determining an expected value in the data stream in response to the perturbation;
comparing the received value to the expected value; and
assessing a validity of the data stream based on the comparing to monitor the data stream.

Example 12. The surgical operating method of any one of examples 10-11, further comprising:
introducing a perturbation to an input signal of the surgical device;
determining an expected control value in response to the perturbation;
determining a difference between the expected control value and a normal control value, wherein the approximation factor associated with the data stream is determined based on the difference between the expected control value and the normal control value; and
adjusting a response of the surgical device based on the approximation factor.

Example 13. The surgical operating method of any one of examples 10-12, wherein detecting that the data stream is invalid further comprises:
obtaining an expected range associated with the measurement; and
determining the data stream comprises a measured value outside of the expected range associated with the measurement.

Example 14. The surgical operating method of any one of examples 10-13, further comprising: generating a corrected data stream associated with the measurement based on the approximation factor and the data stream, wherein the second control signal associated with the surgical device is generated based on the corrected data stream.

Example 15. The surgical operating method of any one of examples 10-14, further comprising: transforming the data stream based on the approximation factor, wherein the second control signal associated with the surgical device is generated based on the transformed data stream.

Example 16. The surgical operating method of any one of examples 10-15, further comprising:
detecting a measurement difference between the data stream and historic data; and
comparing the measurement difference to an error tolerance threshold value, wherein the detecting that the data stream is invalid is based on the measurement difference satisfying the error tolerance threshold value.

Example 17. The surgical operating method of any one of examples 10-16, further comprising:
identifying the surgical device that generates the data stream; and
obtaining an initial characterization of the surgical device, wherein the approximation factor is determined based on the initial characterization of the surgical device.

Example 18. The surgical operating method of any one of examples 10-17, further comprising:
detecting a measurement difference between the data stream and historic data;
identifying a disagreement between the data stream and the historic data; and
determining a cause of the identified disagreement, wherein the approximation factor is determined based on the cause of the identified disagreement.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A surgical system comprising a processor configured to:
obtain a data stream associated with a measurement from a surgical device;
generate a first control signal associated with the surgical device based on the data stream;
detect that the data stream is invalid;
upon detecting that that the data stream is invalid, determine an approximation factor associated with the data stream; and
generate a second control signal associated with the surgical device based on the determined approximation factor.

2. The surgical system of claim 1, wherein the processor is further configured to:
introduce a perturbation to an input signal of the surgical device;
receive a value in the data stream upon introducing the perturbation;
determine an expected value in the data stream in response to the perturbation;
compare the received value to the expected value; and
assess a validity of the data stream based on the comparing to monitor the data stream.

3. The surgical system of claim 1 or claim 2, wherein the processor is further configured to:
introduce a perturbation to an input signal of the surgical device;
determine an expected control value in response to the perturbation;
determine a difference between the expected control value and a normal control value, wherein the approximation factor associated with the data stream is determined based on the difference between the expected control value and the normal control value; and
adjust a response of the surgical device based on the approximation factor.

4. The surgical system of any one of the preceding claims, wherein detecting that the data stream is invalid further comprises:
obtaining an expected range associated with the measurement; and
determining the data stream comprises a measured value outside of the expected range associated with the measurement, wherein the data stream is detected to be invalid based on the data stream comprising a measured value outside of the expected range.

5. The surgical system of any one of the preceding claims, wherein the processor is further configured to:
generate a corrected data stream associated with the measurement based on the approximation factor and the data stream, wherein the second control signal associated with the surgical device is generated based on the corrected data stream.

6. The surgical system of any one of the preceding claims, wherein the processor is further configured to:
transform the data stream based on the approximation factor, wherein the second control signal associated with the surgical device is generated based on the transformed data stream.

7. The surgical system of any one of the preceding claims, wherein the processor is further configured to:
detect a measurement difference between the data stream and historic data; and
compare the measurement difference to an error tolerance threshold value, wherein the detecting that the data stream is invalid is based on the measurement difference satisfying the error tolerance threshold value.

8. The surgical system of any one of the preceding claims wherein determining the approximation factor further comprises:
identifying the surgical device that generates the data stream; and
obtaining an initial characterization of the surgical device, wherein the approximation factor is determined based on the initial characterization of the surgical device.

9. The surgical system of any one of the preceding claims, wherein the processor is further configured to:
detect a measurement difference between the data stream and historic data;
identify a disagreement between the data stream and the historic data; and
determine a cause of the identified disagreement, wherein the approximation factor is determined based on the cause of the identified disagreement.

10. A method performed by a processor in a surgical system, the method comprising:
obtaining a data stream associated with a measurement from a surgical device;
generating a first control signal associated with the surgical device based on the data stream;
detecting that the data stream is invalid;
upon detecting that that the data stream is invalid, determining an approximation factor associated with the data stream; and
generating a second control signal associated with the surgical device based on the determined approximation factor.

11. The method of claim 10, further comprising:
introducing a perturbation to an input signal of the surgical device;
receiving a value in the data stream upon introducing the perturbation;
determining an expected value in the data stream in response to the perturbation;
comparing the received value to the expected value; and
assessing a validity of the data stream based on the comparing to monitor the data stream.

12. The method of claim 10 or claim 11, further comprising:
introducing a perturbation to an input signal of the surgical device;
determining an expected control value in response to the perturbation;
determining a difference between the expected control value and a normal control value, wherein the approximation factor associated with the data stream is determined based on the difference between the expected control value and the normal control value; and
adjusting a response of the surgical device based on the approximation factor.

13. The method of any one of claims 10 to 12, wherein detecting that the data stream is invalid further comprises:
obtaining an expected range associated with the measurement; and
determining the data stream comprises a measured value outside of the expected range associated with the measurement.

14. The method of any one of claims 10 to 13, further comprising:
generating a corrected data stream associated with the measurement based on the approximation factor and the data stream, wherein the second control signal associated with the surgical device is generated based on the corrected data stream.

15. A computer-readable medium comprising instructions which, when executed by a processor, cause the processor to carry out the method of any one of claims 10 to 14.
